# FASCICULE DE BREVET EUROPEEN

(11) **EP 3 972 409 B1**
(45) Date de publication et mention de la délivrance du brevet: **06.12.2023**
(21) Numéro de dépôt: 20725948.2
(22) Date de dépôt: 11.05.2020
(51) Int. Cl.: A01K 67/033, A23K 10/20, A23L 3/36, A23K 40/00, A23K 50/90

(54) **SYSTÈME ET PROCÉDÉ DE PRODUCTION D'INSECTES**
SYSTEM UND VERFAHREN ZUR INSEKTENPRODUKTION
SYSTEM AND METHOD FOR PRODUCING INSECTS

(30) Priorité: 20.05.2019 FR 1905282
(43) Date de publication de la demande: 30.03.2022
(73) Titulaire: Invers, 63720 Saint-Ignat (FR)
(72) Inventeur: CREPIEUX, Sébastien, 63360 Saint-Beauzire (FR); TOURNIER, Ludovic, 63360 Saint-Beauzire (FR); QUITTARD, David, 63360 Saint-Beauzire (FR); CAILLOUX, Stéphanie, 63360 Saint-Beauzire (FR)
(74) Mandataire: Gabriel, Franck
(86) Numéro de dépôt international: PCT/IB2020/054449
(87) Numéro de publication internationale: WO 2020/234688

(56) Documents cités:
- WO-A1-2017/007310
- WO-A2-2019/053439
- FR-A1- 2 871 992
- FR-A1- 3 034 622

## Description

### DOMAINE TECHNIQUE DE L'INVENTION

La présente invention concerne un système et un procédé de production d'insectes, notamment pour l'alimentation animale.

### ETAT DE LA TECHNIQUE ANTERIEURE

Diverses approches existent pour mettre en oeuvre l'élevage d'insectes à grande échelle. Les premières méthodes étaient entièrement manuelles, fastidieuses et avec des taux de productivité peu élevés. Peu à peu, la technicité des équipements utilisés a permis de réduire les interventions humaines dans le processus et d'augmenter la productivité. Aujourd'hui, de nombreux systèmes avec divers équipements technologiques existent.

Par exemple, le document WO2018067376 décrit un système automatisé d'élevage de masse d'insectes. Le système décrit permet de regrouper sur un site de production à grande échelle l'ensemble des étapes de production avec optimisation technique de certaines étapes clés.

Le document WO2012115959 décrit un système d'élevage permettant d'automatiser les phases d'acheminement de nourriture et d'eau pour des bacs d'élevage disposés en colonnes.

Le document FR3034622 décrit un système d'élevage d'insectes pour alimentation animale comportant deux zones, à savoir une Z1 d'élevage comportant une pluralité de bacs d'élevage et une zone Z2 de traitement de ces bacs d'élevage par des postes de travail effectuant diverses opérations sur les bacs. Un système de convoyeur est prévu entre la zone Z1 et la zone Z2 pour effectuer le transfert des bacs entre ces zones. Ainsi, le système décrit comprend un seul site sur lequel l'ensemble des opérations sont regroupées.
Le document FR2871992 décrit un système comparable à celui décrit au document précédent, mais avec une pluralité de bâtiments juxtaposés, agencés côte-à-côte, formant une ferme regroupant l'ensemble des étapes de production.
Les documents WO2017007310 et WO2019053439 décrivent également des systèmes d'élevage d'insectes où les opérations sont toutes effectuées sur un site unique.
Ces systèmes permettent de concentrer divers équipements technologiques dans une usine où l'ensemble des étapes d'élevage et de transformation sont effectuées. Cette approche semble à priori attractive mais présente un certain nombre d'inconvénients. Plus particulièrement, le fait de mettre en place des usines de production à très grande échelle augmente les risques et les conséquences éventuelles en cas de maladie ou de contamination. Les quantités d'énergies requises sont importantes. Les rejets tels que les excréments des insectes en cours de croissance doivent être évacués sur d'autres sites, engendrant des coûts élevés et des sources de pollution. La nourriture utilisée pour assurer la croissance des insectes est acheminée depuis des sources d'approvisionnement externes, là encore avec des coûts importants et des sources de pollution.
On constate que les système et procédés récemment développés ont été conçus dans un but premier de simplifier et d'optimiser les nombreuses manipulations à effectuer afin de réduire les besoins d'interventions humaines, sans prendre en compte le contexte plus global de l'ensemble des ressources utiles.
Il existe donc un besoin pour un dispositif et un procédé qui permet d'optimiser l'utilisation des ressources utiles pour produire des insectes, de préférence en maximisant la circularité des échanges et des bénéfices réciproques, sans entrer en compétition avec des ressources pour l'alimentation humaine et engendrant une faible pression sur les surfaces agricoles.
Pour pallier ces différents inconvénients, l'invention prévoit différents moyens techniques.

### EXPOSE DE L'INVENTION

Tout d'abord, un premier objectif de l'invention consiste à fournir un système et un procédé de production de produits finis ou semi-finis à base d'insectes.

Un autre objectif de l'invention consiste à fournir un système et un procédé de production de produits finis ou semi-finis à base d'insectes entiers.

Encore un objectif de l'invention consiste à fournir un système et un procédé de production de produits alimentaires à base d'insectes pour l'alimentation animale.

Pour ce faire, l'invention prévoit système de production d'insectes entiers, notamment pour production de nourriture pour animaux, comportant une pluralité de micro-stations de production d'insectes, chacune des micro-stations de production étant conçue pour assurer de façon indépendante une phase de croissance des larves et comprend un module d'élevage local comprenant une pluralité de bacs de croissance empilables et un ilot de traitement robotisé, adapté pour récupérer des bacs de croissance d'une part des matières organiques à utiliser pour engrais local et d'autre part les insectes ayant atteints une taille minimale prédéfinie pour transformation en produits finis ou semi-finis, dans lequel :
- le système de production comporte par ailleurs une centrale unique de transformation en produits finis ou semi-finis à base d'insectes seuls ou d'insectes combinés avec d'autres ingrédients, ladite centrale unique de transformation étant située sur un site à distance des micro-stations et en relation fonctionnelle avec ces dernières, et comprenant:
   a) un module de réception d'insectes froids en provenance des micro-stations assurant le maintien d'une chaîne du froid préalablement amorcée dans les micro-stations d'origine des insectes;
   b) un module de transformation de lots d'insectes refroidis en produits finis ou semi-finis;
   c) un module d'élevage de reproducteurs ;
- chacune des micro-stations comprends un module de refroidissement conçu pour refroidir les insectes avant expédition vers la centrale unique de transformation;
- la le site de la centrale unique ainsi que chacun des sites des micro-stations sont situés les uns par rapport aux autres à une distance de sécurité (SD) d'au moins 2 km et plus préférentiellement au moins 5 km;
- et les micro-stations sont reliées à la centrale unique de transformation d'une part par une chaîne d'approvisionnement de lots de reproducteurs et/ou d'oeufs conçue pour acheminer des lots de reproducteurs et/ou des oeufs depuis la centrale de transformation vers au moins une partie des micro-stations, et d'autre part par une chaîne du froid conçue pour acheminer les insectes refroidis des micro-stations vers la centrale de transformation.

Une telle architecture permet de réduire le risque et les inconvénients liés aux maladies ou à un éventuel problème de contamination. Sur un site de taille petite ou moyenne, il est plus facile d'isoler les candidats atteints de maladie ou infection et de traiter le site que sur un site d'élevage à très grands volumes. En cas de perte d'exploitation, les pertes sont restreintes à une seule micro-station. L'architecture avec un site centralisé spécialisé et une pluralité de micro-stations permet d'optimiser les ressources de chaque endroit. Les opérations très spécialisées ou nécessitant soit une expertise spécifique et/ou des équipements onéreux sont centralisés et peuvent ainsi bénéficier à plusieurs sites de production.

Selon un mode de réalisation avantageux, l'îlot de traitement robotisé comprend au moins un module de séparation conçu pour effectuer une extraction de matières organiques (par exemple les excréments et déchets des insectes) des bacs de croissance, pour utilisation comme fertilisant local sur la micro-station, et pour récupérer les insectes à refroidir pour expédition à la station unique de transformation.

Selon une variante avantageuse, au moins une partie des micro-stations comportent un module de production de cultures végétales dédiées à l'alimentation locale des insectes produits dans la même micro-station. Les micro-stations peuvent de la sorte permettre une optimisation des tâches et établir un cercle vertueux entre les cultures effectuées sur place pour nourrir les insectes, et l'utilisation des rejets des insectes pour nourrir les sols cultivés de la micro-station.

Selon encore un mode de réalisation avantageux, les produits finis ou semi-finis sont des produits alimentaires déshydratés, notamment pour alimentation animale.

Le système de production peut également prévoir un module de logistique conçu pour envoyer des lots de produits finis ou semi-finis vers des sites de transformation ou de consommation. De préférence, chaque micro-station est située à une distance inférieure à 100 km de la centrale unique.

L'invention prévoit également un procédé de production d'insectes entiers, notamment pour production de nourriture pour animaux, pour système de production d'insectes entiers tel que préalablement décrit, ledit procédé comprenant les étapes suivantes:
i) dans la centrale unique, une étape de production de lots d'insectes reproducteurs;
ii) une phase de pontes d'oeufs par les insectes reproducteurs effectuée en centrale unique et/ou en micro-station;
iii) par chacune des micro-stations de production, chacune situées sur un site spécifique, distants entre eux et en regard du site de la centrale unique d'une distance de sécurité (SD) d'au moins 2 km et plus préférentiellement au moins 5 km; une étape de croissance des larves issues des oeufs dans des bacs de production ;
iv) par chacune des micro-stations de production, au moins une étape d'ajout de formulation alimentaire de croissance issue au moins en partie de cultures localisées sur la même micro-station ;
v) par chacune des micro-stations de production, une étape de récupération des insectes protéinés produits localement par un module de séparation;
vi) par chacune des micro-stations de production, une étape de refroidissement des insectes produits localement;
vii) par chacune des micro-stations de production, une étape d'amorçage d'une chaîne du froid et de mise en place des insectes refroidis dans des modules de transport par lots pour acheminement à la centrale unique associée;
viii) dans la centrale unique, sur un site à distance des micro-stations d'une distance de sécurité (SD) d'au moins 2 km et plus préférentiellement au moins 5 km, une étape de réception des lots en provenance de la pluralité de micro-stations de production en assurant le suivi de la chaîne du froid amorcée dans chacune des micro-stations ;
ix) dans la centrale unique, une étape de transformation des lots d'insectes entiers refroidis en produits finis ou semi-finis.

De manière avantageuse, l'étape de transformation en produits finis ou semi-finis comprend une phase de déshydratation. La déshydratation est effectuée par exemple dans une enceinte chauffante ou à micro-ondes ou pendant une phase de mise en forme par extrusion.

Les produits finis ou semi-finis fabriqués comportent soit des insectes seuls, ou des insectes en combinaison avec d'autres ingrédients, par exemple sous forme de granulés ou croquettes. Les produits déshydratés obtenus facilitent la conservation, le stockage et la manipulation. Grâce à un tel procédé, chaque site est spécialisé et optimisé, pour une productivité élevée, et une optimisation des ressources locales, tout en réduisant les besoins de transport. La nourriture produite comporte de grandes qualités nutritives et convient à des usages multiples, tant pour l'élevage d'animaux que pour l'alimentation quotidienne des animaux domestiques.

De manière avantageuse, le procédé est utilisé pour produire des coléoptères, plus préférentiellement des tenebrio, et encore plus préférentiellement des tenebrio molitor. Ces types d'insectes sont particulièrement bien adaptés à ce type de procédé. Ils comportent par ailleurs de grandes qualités nutritives, dont un taux élevé de protéines.

Selon un mode de réalisation avantageux, le précédé prévoit, pour au moins une partie des micro-stations, une étape de production de cultures végétales dédiées à l'alimentation locale des insectes produits dans la même micro-station. Cette étape permet d'optimiser les ressources de la micro-station et de réduire les besoins de transport.

Selon encore un mode de réalisation avantageux, le procédé prévoit, pour au moins une partie des micro-stations de production, une étape de séparation des insectes reproducteurs des oeufs permettant de conserver uniquement les oeufs dans les bacs de production.

Selon un autre mode de réalisation avantageux, le procédé prévoit, pour au moins une partie des micro-stations de production, au moins une étape d'extraction des bacs de matières organiques (par exemple des excréments et déchets d'insectes) pour utilisation comme fertilisant sur la même micro-station.

Cette étape permet de bénéficier sur site des bienfaits des résidus de production des insectes pour obtenir des engrais et amendements naturels et organiques de bonne qualité.

De manière avantageuse, les insectes produits sont au stade larvaire. En effet, c'est le stade de croissance permettant l'obtention d'un taux élevé de protéines, tout en conservant des cycles de production relativement courts.

Par « insectes entiers » on entend des insectes à l'état larvaire ou adulte non réduits en poudre ou farine et non liquéfiés. Les insectes sont 100% entiers, soit en morceaux.

### DESCRIPTION DES FIGURES

Tous les détails de réalisation sont donnés dans la description qui suit, complétée par les figures 1 à 3, présentées uniquement à des fins d'exemples non limitatifs, et dans lesquelles:
- la figure 1 est une représentation schématique illustrant le fonctionnement et les flux mis en oeuvre pour un exemple de système de production ;
- la figure 2 est une représentation schématique d'un exemple de système de production comportant une centrale de transformation avec différents modules et une pluralité de micro-stations avec leurs modules spécifiques;
- la figure 3 est un organigramme fonctionnel d'un exemple de procédé de production d'insectes.

### DESCRIPTION DETAILLEE DE L'INVENTION

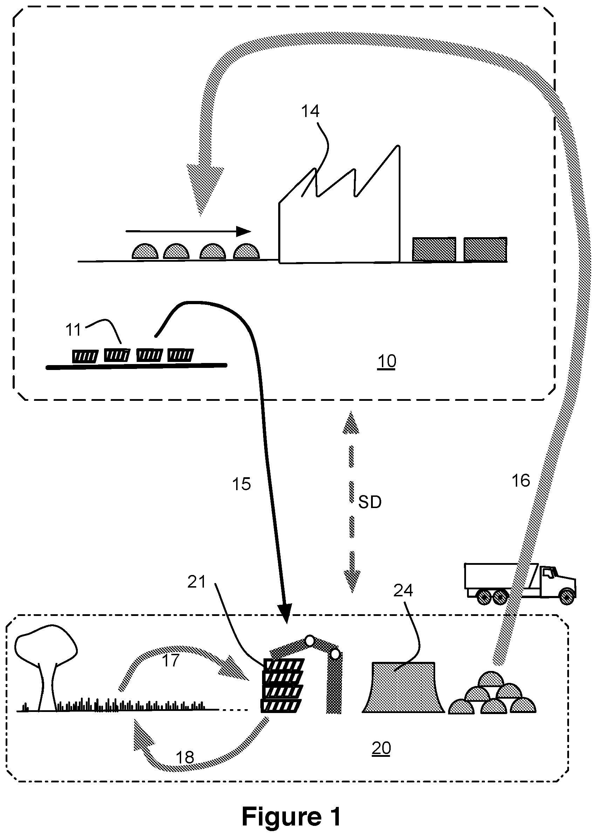

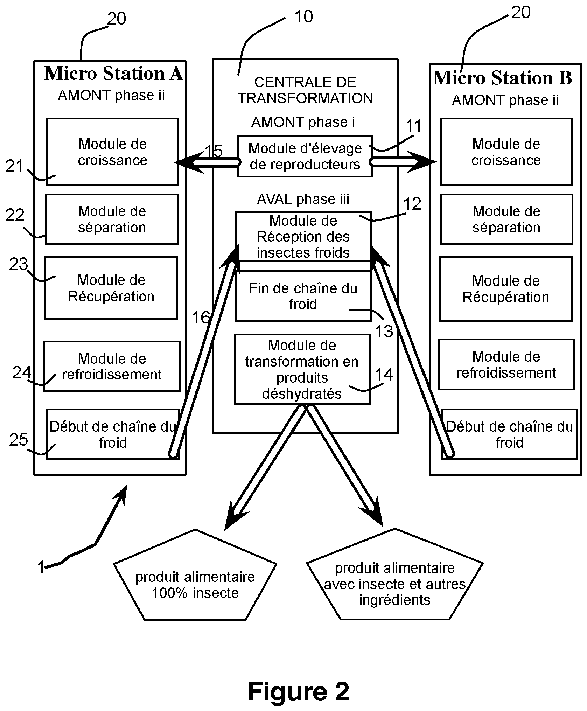

**Les** figures 1 et 2 sont des représentations schématiques d'un exemple de système de production d'insectes, notamment pour produire de la nourriture pour animaux à base d'insectes entiers. Tel qu'illustré, le système comporte une centrale unique 10 de transformation en relation fonctionnelle avec une pluralité de micro-stations 20 de production d'insectes réparties dans une zone géographique donnée par exemple autour de la centrale unique 10.

**La** centrale unique 10 de transformation est située sur un site à distance des micro-stations 20 afin d'assurer une distance de sécurité SD entre le site de la centrale unique et chacun des sites des micro-stations 20. Pour la même raison, les micro-stations 20 sont chacune situées sur un site spécifique, distants entre eux et en regard du site de la centrale unique. Le site de la centrale unique 10 ainsi que les sites de chacune des micro-stations sont situés les uns par rapport aux autres à une distance de sécurité (SD) d'au moins 2 km et plus préférentiellement au moins 5 km. Grâce à cette distance de sécurité, le système et le procédé de production d'insectes permettent d'assurer la sécurité sanitaire d'un système de production sans risque d'interruption de la production, tout en optimisant les coûts grâce à un groupement de certaines opérations clés du système de production. Si une micro-station subit un arrêt de production du fait d'une maladie, contamination, ou autre, les autres micro-stations ainsi que la station centrale unique sont préservées et peuvent assurer la continuité de l'approvisionnement alimentaire. L'approvisionnement de la centrale unique avec une chaîne du froid tel que décrit ci-après contribue à assurer la sécurité sanitaire.

Chaque micro-station 20 de production comprend une pluralité de modules permettant de mettre en oeuvre des phases de croissance des insectes, au moins une phase de séparation et une phase de refroidissement, avec l'amorce d'une chaîne du froid 16. On retrouve ainsi au moins un module 21 d'élevage local comprenant une pluralité des bacs de croissance empilables dans lesquels sont placés les oeufs permettant d'amorcer la phase d'élevage. Divers nutriments et de l'eau sont également placés dans le bac afin de nourrir les larves grandissantes.

Pour effectuer les principales manipulations en relation avec les bacs, un ilot de traitement robotisé est prévu. Cet ilot robotisé est conçu en outre pour extraire des bacs de croissance des matières organiques tels que les excréments des larves. Ces matières sont ensuite disponibles pour utilisation en tant qu'engrais pour les cultures locales effectuées au niveau de la micro-station. L'ilot permet également de récupérer les insectes ayant atteints une taille minimale prédéfinie pour transformation en produits finis ou semi-finis. Le présent système et le procédé de production correspondant ultérieurement décrit sont avantageusement conçus pour une récupération des insectes au stade larvaire. La micro-station comprend par ailleurs un module 24 de refroidissement, conçu pour refroidir les insectes, de préférence avec un effet de refroidissement instantané ou quasi-instantané pour éviter que les insectes subissent un stress.

**De** son côté, la centrale unique 10 de transformation comprend un module utile en amont du processus de production, et des modules utilisés en fin de cycle, lorsque le cycle de croissance des insectes est achevé. Ainsi, on retrouve tout d'abord un module 11 d'élevage d'insectes reproducteurs, qui permettront de produire les oeufs amorçant un cycle de croissance d'insectes. On retrouve ensuite un module 12 de réception d'insectes froids assurant le maintien d'une chaîne du froid préalablement amorcée dans les micro-stations et permettant de recevoir les insectes préalablement refroidis à une micro-station.

**Un** module 14 de transformation de lots d'insectes refroidis en produits finis ou semi-finis déshydratés permet de traiter les insectes par un processus de déshydratation susceptible de produire par exemple un produit alimentaire constitué à 100 % ou presque d'insectes, ou une produit alimentaire comportant des insectes et d'autres ingrédients. Dans le premier cas, le produit alimentaire résultant consiste par exemple en insectes entiers séchés ou déshydratés. Dans le second cas, le produit est réalisé sous forme de croquettes ou granules ou grains. La centralisation des moyens de transformation en produits finis ou semi-finis au niveau du site de la centrale unique à une distance de sécurité (SD) des autres sites et approvisionnée par une chaîne du froid procure un double niveau de sécurité afin de garantir la pérennité de la chaîne de production alimentaire.

Tel qu'illustré, les micro-stations 20 sont reliées à la centrale unique de transformation en amont par une chaîne d'approvisionnement 16 de lots de reproducteurs ou d'oeufs conçue pour acheminer les lots de reproducteurs ou les oeufs pondus par ces reproducteurs de la centrale 10 de transformation vers au moins une partie des micro-stations 20. En aval, les micro-stations 20 sont reliées à la centrale 10 par une chaîne du froid 16 conçue pour acheminer les insectes refroidis des micro-stations 20 vers la centrale 10 de transformation. On utilise par exemple des camions réfrigérés faisant la navette entre les micro- stations et la centrale. Tel qu'illustré à la figure 2, au moins une partie des micro-stations 20 comportent un module de production de cultures végétales dédiées à l'alimentation locale des insectes produits dans la même micro-station.

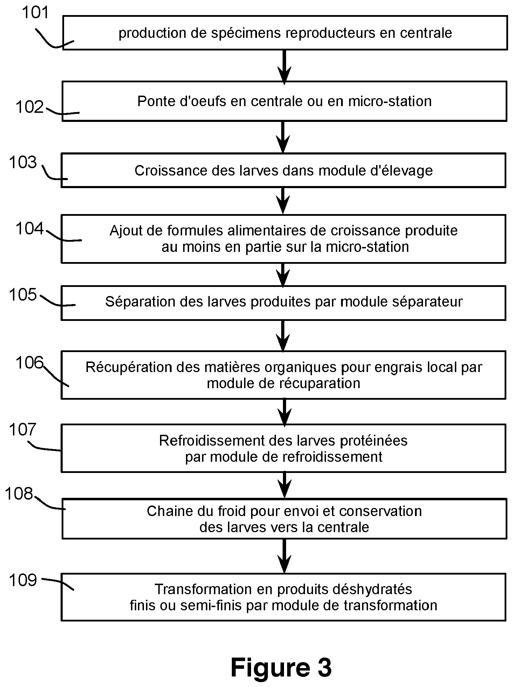

La figure 3 est un organigramme fonctionnel illustrant les principales étapes d'un exemple de procédé de production d'insectes. Ce procédé fonctionne à l'aide d'un système 1 de production comportant une centrale unique 10 de transformation qui est en relation fonctionnelle avec une pluralité de micro-stations 20 de production prévues à proximité ou à une distance raisonnable de la centrale unique. Tel qu'illustré, le procédé prévoit, pour amorcer un cycle de production, une étape 101 de production de lots d'insectes reproducteurs effectuée en centrale unique. Cette phase permet de fournir, pour chaque micro-station 20, les insectes reproducteurs utiles pour effectuer les pontes, à l'étape 102, permettant d'amorcer un cycle de production. Après la ponte, pour récupérer les insectes reproducteurs, le procédé prévoit avantageusement une étape de séparation des insectes reproducteurs des oeufs permettant de conserver uniquement les oeufs dans les bacs de production.

En variante, les reproducteurs pondent les oeufs en central unique, et ces derniers sont ensuite expédiés aux diverses micro-stations.

La phase de croissance 103 des larves peut ensuite démarrer. Chaque micro-station gère cette phase de façon indépendante, en utilisant de préférence les ressources locales. Ainsi par exemple, l'étape 104 prévoit une ou plusieurs phases d'ajout de formulation alimentaire de croissance issue de la micro-station où les insectes sont en cours de croissance. On optimise ainsi l'utilisation des ressources locales, en réduisant les besoins de transport, et en contribuant à minimiser les sources de pollution. Par exemple, des cultures végétales dédiées à l'alimentation locale des insectes produits dans la micro-station sont effectuées au niveau de la micro-station, tel qu'illustré à la figure 1 par la flèche 17.

Toujours pour optimiser l'utilisation des ressources locales, on prévoit une ou plusieurs étapes optionnelles 106 de récupération de matières organiques pour utilisation comme fertilisant sur la même micro-station. Cette étape est illustrée par la flèche 18 de la figure 1. Lorsque les insectes ont atteint le stade de croissance souhaité, le procédé prévoit une étape 105 de récupération des insectes protéinés produits localement.

Ensuite, à l'étape 107, les insectes entiers récupérés subissent une phase de refroidissement. Pour minimiser les sources de stress, le refroidissement est de préférence mis en oeuvre le plus rapidement possible, par exemple pour endormir les insectes, et/ou les congeler. On utilise par exemple des enceintes de refroidissement tel que des bacs cryogéniques. Le refroidissement peut être précédé ou adjoint en simultané d'une mise en ambiance gazeuse, de préférence de CO2, permettant d'accélérer cette phase et de réduire d'autant les risques de stress.

**Une** chaîne du froid 16 est ensuite amorcée à l'étape 108 pour faire en sorte de maintenir les insectes refroidis à une température désirée, en mode congélation ou en simple refroidissement. A l'arrivée de la chaine du froid 16, à la central de transformation unique 10 associée, les lots d'insectes en provenances des micro-stations sont récupérés. On prévoit de préférence une traçabilité afin d'identifier les lots, leur provenance, etc.

Il est à noter qu'à ce stade, au niveau de la centrale de transformation unique, les insectes sont toujours entiers et de préférence au stade larvaire. Ils peuvent alors subir une ou plusieurs étapes 109 de transformation en produit alimentaire. La transformation implique une phase de déshydratation, afin que le produit alimentaire obtenu soit facile à conserver et à manutentionner. Les produits alimentaires obtenus sont donc de préférence en format non farineux et non liquide ou laiteux.

### Numéros de référence employés sur les figures

| | |
|---|---|
| 1 | Système de production de nourriture |
| 2 | -- |
| 3 | -- |
| 4 | -- |
| 5 | -- |
| 6 | -- |
| 7 | -- |
| 8 | -- |
| 9 | -- |
| 10 | Centrale de transformation unique |
| 11 | Module d'élevage de reproducteurs |
| 12 | Module de réception des insectes froids |
| 13 | Arrivée de la chaîne du froid |
| 14 | Module de transformation en produits déshydratés |
| 15 | Chaîne d'approvisionnement de reproducteurs et/ou d'oeufs |
| 16 | Chaîne du froid |
| 17 | Alimentation avec source locale |
| 18 | Utilisation locale d'engrais obtenus par les rejets de production |
| 19 | -- |
| 20 | Micro-station |
| 21 | Module de croissance |
| 22 | Module de séparation |
| 23 | Module de récupération |
| 24 | Module de refroidissement |
| | |

## Revendications

1. Système (1) de production d'insectes entiers, notamment pour production de nourriture pour animaux, comportant une pluralité de micro-stations (20) de production d'insectes, chacune des micro-stations de production étant conçue pour assurer de façon indépendante une phase de croissance des larves et comprend un module (21) d'élevage local comprenant une pluralité de bacs de croissance empilables et un ilot de traitement robotisé, adapté pour récupérer des bacs de croissance d'une part des matières organiques à utiliser pour engrais local et d'autre part les insectes ayant atteints une taille minimale prédéfinie pour transformation en produits finis ou semi-finis;
**caractérisé:**
- **en ce que** le système de production comporte par ailleurs une centrale unique (10) de transformation en produits finis ou semi-finis à base d'insectes seuls ou d'insectes combinés avec d'autres ingrédients, ladite centrale unique de transformation étant située sur un site à distance des micro-stations et en relation fonctionnelle avec ces dernières, et comprenant:
a) un module (12) de réception d'insectes froids en provenance des micro-stations assurant le maintien d'une chaîne du froid préalablement amorcée dans les micro-stations d'origine des insectes;
b) un module (14) de transformation de lots d'insectes refroidis en produits finis ou semi-finis;
c) un module (11) d'élevage de reproducteurs ;
- **en ce que** chacune des micro-stations comprends un module (24) de refroidissement conçu pour refroidir les insectes avant expédition vers la centrale unique (10) de transformation ;
- **en ce que** le site de la centrale unique (10) ainsi que chacun des sites des micro-stations sont situés les uns par rapport aux autres à une distance de sécurité (SD) d'au moins 2 km et plus préférentiellement au moins 5 km;
- et **en ce que** les micro-stations sont reliées à la centrale unique de transformation d'une part par une chaîne d'approvisionnement (15) de lots de reproducteurs et/ou d'oeufs conçue pour acheminer des lots de reproducteurs et/ou des oeufs depuis la centrale (10) de transformation vers au moins une partie des micro-stations (20), et d'autre part par une chaîne du froid (16) conçue pour acheminer les insectes refroidis des micro-stations (20) vers la centrale (10) de transformation.

2. Système de production selon la revendication 1, dans lequel l'îlot de traitement robotisé comprend au moins un module de séparation (22) conçu pour effectuer une extraction de matières organiques des bacs de croissance, pour utilisation comme fertilisant local sur la micro-station, et pour récupérer les insectes à refroidir pour expédition à la station unique (10) de transformation.

3. Système de production selon la revendication 1, dans lequel au moins une partie des micro-stations (20) comportent un module de production de cultures végétales dédiées à l'alimentation locale des insectes produits dans la même micro-station.

4. Système de production selon l'une quelconque des revendications 1 ou 2, dans lequel les produits finis ou semi-finis sont des produits alimentaires déshydratés, notamment pour alimentation animale.

5. Procédé de production d'insectes entiers, notamment pour production de nourriture pour animaux, pour système de production d'insectes entiers selon l'une quelconque des revendications 1 à 4, ledit procédé comprenant les étapes suivantes :
i) dans la centrale unique (10), une étape (101) de production de lots d'insectes reproducteurs;
ii) une phase (102) de pontes d'oeufs par les insectes reproducteurs effectuée en centrale unique (10) et/ou en micro-station (20) ;
iii) par chacune des micro-stations (20) de production, chacune situées sur un site spécifique, distants entre eux et en regard du site de la centrale unique (10) d'une distance de sécurité (SD) d'au moins 2 km et plus préférentiellement au moins 5 km, une étape (103) de croissance des larves issues des oeufs dans des bacs de production ;
iv) par chacune des micro-stations (20) de production, au moins une étape (104) d'ajout de formulation alimentaire de croissance issue au moins en partie de cultures localisées sur la même micro-station ;
v) par chacune des micro-stations (20) de production, une étape (105) de récupération des insectes protéinés produits localement par un module de séparation;
vi) par chacune des micro-stations (20) de production, une étape (107) de refroidissement des insectes produits localement;
vii) par chacune des micro-stations (20) de production, une étape (108) d'amorçage d'une chaîne du froid et de mise en place des insectes refroidis dans des modules de transport par lots pour acheminement à la centrale unique associée (10);
viii) dans la centrale unique (10), sur un site à distance des micro-stations (20) d'une distance de sécurité (SD) d'au moins 2 km et plus préférentiellement au moins 5 km, une étape de réception des lots en provenance de la pluralité de micro-stations (20) de production en assurant le suivi de la chaîne du froid amorcée dans chacune des micro-stations ;
ix) dans la centrale unique (10), une étape (109) de transformation des lots d'insectes entiers refroidis en produits finis ou semi-finis.

6. Procédé de production selon la revendication 5, dans lequel l'étape (109) de transformation en produits finis ou semi-finis comprend une phase de déshydratation.

7. Procédé de production selon la revendication 6, dans lequel la phase de déshydratation est effectuée dans une enceinte chauffante ou à micro-ondes ou pendant une phase de mise en forme par extrusion.

8. Procédé de production selon l'une quelconque des revendications 5 à 7, comprenant, pour au moins une partie des micro-stations de production, au moins une étape (106) d'extraction des bacs de matières organiques pour utilisation comme fertilisant sur la même micro-station.

9. Procédé de production selon l'une quelconque des revendications 5 à 8, dans lequel les insectes produits sont au stade larvaire.

## Patentansprüche

1. Produktionssystem (1) ganzer Insekten, insbesondere zur Herstellung von Tierfutter, das eine Vielzahl von Mikrostationen (20) zur Produktion von Insekten umfasst, wobei jede der Produktionsmikrostationen dazu ausgelegt ist, unabhängig eine Wachstumsphase der Larven sicherzustellen, und ein Modul (21) zur lokalen Aufzucht umfasst, das eine Vielzahl stapelbarer Aufzuchtbehälter und eine robotergesteuerte Verarbeitungsinsel umfasst, die dazu angepasst ist, aus den Wachstumsbehältern einerseits organische Stoffe zur Verwendung als lokales Düngemittel und andererseits Insekten, die eine vordefinierte Mindestgröße erreicht haben, zur Transformation in End- oder Halbfertigprodukte zu gewinnen;
**dadurch gekennzeichnet:**
- **dass** das Produktionssystem außerdem eine einzige Zentrale (10) zur Transformation in End- oder Halbfertigprodukte basierend auf Insekten allein oder Insekten kombiniert mit anderen Bestandteilen umfasst, wobei sich die einzige Transformationszentrale auf einem von den Mikrostationen entfernten Standort und in Funktionsbeziehung mit diesen Letzteren befindet und Folgendes umfasst:
a) ein Aufnahmemodul (12) kalter Insekten, die aus den Mikrostationen kommen, das das Aufrechterhalten einer Kältekette, die zuvor in den Ursprungsmikrostationen der Insekten eingeleitet wurde, sicherstellt;
b) ein Modul (14) zur Transformation von Chargen gekühlter Insekten in End-oder Halbfertigprodukte;
c) ein Modul (11) zur Aufzucht von Zuchttieren;
- **dass** jede der Mikrostationen ein Kühlmodul (24) umfasst, das dazu ausgelegt ist, die Insekten vor dem Versand zu der einzigen Transformationszentrale (10) zu kühlen;
- **dass** sich der Standort der einzigen Zentrale (10) sowie jeder der Mikrostationenstandorte zueinander in einer Sicherheitsentfernung (SD) von mindestens 2 km und bevorzugter mindestens 5 km befinden;
- und **dass** die Mikrostationen mit der einzigen Transformationszentrale einerseits durch eine Versorgungskette (15) von Zuchttier- und/oder Eierchargen verbunden sind, die dazu ausgelegt ist, die Zuchttier- und/oder Eierchargen von der Transformationszentrale (10) zu mindestens einem Teil der Mikrostationen (20) zu befördern, und andererseits durch eine Kältekette (16), die dazu ausgelegt ist, die gekühlten Insekten der Mikrostationen (20) zu der Transformationszentrale (10) zu befördern.

2. Produktionssystem nach Anspruch 1, wobei die robotisierte Verarbeitungsinsel mindestens ein Trennmodul (22) umfasst, das dazu ausgelegt ist, eine Extraktion organischer Stoffe aus den Wachstumsbehältern zur Verwendung als lokales Düngemittel auf der Mikrostation auszuführen, und um die zu kühlenden Insekten zum Versand an die einzige Transformationsstation (10) zu gewinnen.

3. Produktionssystem nach Anspruch 1, wobei mindestens ein Teil der Mikrostationen (20) ein Produktionsmodul von Pflanzenkulturen umfasst, die zur lokalen Versorgung der produzierten Insekten in derselben Mikrostation bestimmt sind.

4. Produktionssystem nach einem der Ansprüche 1 oder 2, wobei die End-oder Halbfertigprodukte getrocknete Nahrungsmittel, insbesondere für Tierfutter, sind.

5. Produktionsverfahren ganzer Insekten, insbesondere zur Nahrungsmittelproduktion für Tiere, für ein Produktionssystem ganzer Insekten nach einem der Ansprüche 1 bis 4, wobei das Verfahren die folgenden Schritte umfasst:
i) in der einzigen Zentrale (10), einen Produktionsschritt (101) von Zuchttierchargen;
ii) eine Eierlegephase (102) durch die Zuchtinsekten, die in der einzigen Zentrale (10) und/oder in einer Mikrostation (20) ausgeführt wird;
iii) durch jede der Produktionsmikrostationen (20), die sich jeweils auf einem spezifischen Standort befinden, die voneinander und gegenüber dem Standort der einzigen Zentrale (10) um eine Sicherheitsentfernung (SD) von mindestens 2 km und bevorzugter mindestens 5 km entfernt liegen, einen Wachstumsschritt (103) der Larven, die aus den Eiern, in den Produktionsbehältern hervorgehen
iv) durch jede der Produktionsmikrostationen (20) mindestens einen Zugabeschritt (104) von Wachstums-Nahrungsrezeptur, die mindestens zum Teil aus Kulturen stammt, die sich auf derselben Mikrostation befinden;
v) durch jede der Produktionsmikrostationen (20) einen Gewinnungsschritt (105) der lokal produzierten Proteininsekten, durch ein Trennmodul;
vi) durch jede der Produktionsmikrostationen (20) einen Kühlschritt (107) der lokal produzierten Insekten;
vii) durch jede der Produktionsmikrostationen (20) einen Einleitungsschritt (108) der Kältekette und Anordnung der gekühlten Insekten in Transportmodulen in Chargen zur Beförderung zu der einzigen zugeordneten Zentrale (10);
viii) in der einzigen Zentrale (10), auf einem von den Mikrostationen (20) um eine Sicherheitsentfernung (SD) von mindestens 2 km und bevorzugter mindestens 5 km entfernten Standort, einen Abnahmeschritt der Chargen, die von der Vielzahl von Produktionsmikrostationen (20) stammen, unter Sicherstellen der Verfolgung der in jeder der Mikrostationen eingeleiteten Kältekette;
ix) in der einzigen Zentrale (10), einen Transformationsschritt (109) der Chargen ganzer gekühlter Insekten in End- oder Halbfertigprodukte.

6. Produktionsverfahren nach Anspruch 5, wobei der Transformationsschritt (109) in End- oder Halbfertigprodukte eine Trocknungsphase umfasst.

7. Produktionsverfahren nach Anspruch 6, wobei die Trocknungsphase in einem Heiz- oder Mikrowelleneinschluss oder während der Extrusionsformungsphase ausgeführt wird.

8. Produktionsverfahren nach einem der Ansprüche 5 bis 7, das für mindestens einen Teil der Produktionsmikrostationen mindestens einen Extraktionsschritt (106) organischer Stoffe aus den Behältern zur Verwendung als Düngemittel auf derselben Mikrostation umfasst.

9. Produktionsverfahren nach einem der Ansprüche 5 bis 8, wobei sich die produzierten Insekten im Larvenstadium befinden.

## Claims

1. System (1) for the production of whole insects, in particular for animal feed, comprising a plurality of insect production micro-stations (20), each production micro-station being designed to independently carry out a larva growth phase and comprising a local rearing module (21) with a plurality of stackable rearing trays and a robotised processing island adapted to collect from the rearing trays, on the one hand organic material for use as local fertilizer and on the other hand insects that have reached a pre-defined minimum size for transformation into finished or semi-finished products;
**characterised:**
- **in that** the production system further comprises a single central transformation station (10) for finished or semi-finished products based on insects alone or insects combined with other ingredients, said single central transformation station being located on a site remote from the micro-stations and in functional relation with the latter, and comprising:
a) a reception module (12) for chilled insects from the micro-stations, providing a maintained cold chain previously initiated in the original insect micro-stations;
b) a transformation module (14) for batches of chilled insects as finished or semi-finished products;
c) a rearing module (11) for reproducers;
- **in that** each of the micro-stations comprises a chilling module (24) designed to cool the insects before dispatch to the single central transformation station (10);
- **in that** the single central station site (10) together with each of the micro-station sites are located at a safety distance (SD) from each other of at least 2 km and more preferentially 5 km;
- and **in that** the micro-stations are linked to the single central transformation station on the one hand by a supply chain (15) for batches of reproducers and/or eggs designed to convey batches of reproducers and/or eggs from the transformation station (10) to at least a part of the micro-stations (20), and on the other hand by a cold chain (16) designed to convey the chilled insects from the micro-stations (20) to the central transformation station (10).

2. Production system according to claim 1 wherein the robotised processing island comprises at least one separating module (22) designed to extract organic material (for example insect excrement and waste) from the rearing trays, for use as local fertilizer on the micro-station, and to collect the insects for chilling for dispatch to the single transformation station (10).

3. Production system according to claim 1 wherein at least part of the micro-stations (20) comprises a module for the production of plant crops for locally feeding the insects produced in the same micro-station.

4. Production system according to any one of claims 1 or 2 wherein the finished or semi-finished products are dehydrated food products, in particular for animal feed.

5. Production process for whole insects, in particular for the production of animal feed for a whole insect production system according to any one of claims 1 to 4, said process comprising the following steps:
i) in the single central station (10), a step (101) of production of batches of reproducing insects;
ii) a phase (102) of egg laying by the reproducing insects in a single central station (10) and/or in a micro-station (20);
iii) for each of the production micro-stations (20), each located on a specific site, separated by a safety distance (SD) from the single central station (10) of at least 2 km and more preferentially at least 5 km, a step (103) of growth of larvae from eggs in the production trays;
iv) for each production micro-station (20), at least one step (104) of adding a food growth formulation from at least part of the crops located at the same micro-station;
v) for each production micro-station (20), a step (105) of collecting protein-rich insects locally by a separating module;
vi) for each production micro-station (20), a step (107) of chilling the locally produced insects;
vii) for each production micro-station (20), a step (108) of initiating a cold chain and placing chilled insects in transport modules for conveying to the associated single central station (10);
viii) in the single central station (10), on a site at a safety distance (SD) from the micro-stations (20) of at least 2 km, more preferentially at least 5 km, a step of reception of batches from the plurality of production micro-stations (20), with the cold chain initiated in each of the micro-stations being maintained;
ix) in the single central station (10), a step (109) of transformation of batches of whole chilled insects into finished or semi-finished products.

6. Production process according to claim 5, wherein the step (109) of transformation into finished or semi-finished products comprises a dehydration phase.

7. Production process according to claim 6 wherein the dehydration phase is carried out in a heated or microwave enclosure or during a phase of forming by extrusion.

8. Production process according to any one of claims 5 to 7, comprising, for at least part of the production micro-stations, at least a step (106) of extraction of organic material for use as fertilizer on the same micro-station.

9. Production process according to any one of claims 5 to 8, wherein the insects produced are at the larval stage.
